# EUROPÄISCHE PATENTSCHRIFT

(11) **EP 2 377 830 B1**
(45) Veröffentlichungstag und Bekanntmachung des Hinweises auf die Patenterteilung: **13.04.2016**
(21) Anmeldenummer: 10168792.9
(22) Anmeldetag: 07.07.2010
(51) Int. Cl.: C03C 10/12, C03C 3/095, A61K 6/027, C04B 41/50, C04B 41/85

(54) **Lithiumsilikat-Glaskeramik und -Glas mit Übergangsmetalloxid**
Lithium silicate glass ceramic and glass with transition metal oxide
Vitrocéramique et verre à base du silicate de lithium ayant d'oxyde de métal de transition

(30) Priorität: 16.04.2010 EP 10160222
(43) Veröffentlichungstag der Anmeldung: 19.10.2011
(73) Patentinhaber: Ivoclar Vivadent AG, 9494 Schaan (LI)
(72) Erfinder: Ritzberger, Christian, 9472 Grabs (CH); Höland, Wolfram, 9494 Schaan (LI); Schweiger, Marcel, 7000 Chur (CH); Rheinberger, Volker, 9490 Vaduz (CH)
(74) Vertreter: Uexküll & Stolberg

(56) Entgegenhaltungen:
- EP-A1- 0 690 031
- WO-A1-2009/126317
- DE-A1-102005 026 269
- US-A1- 2002 009 600
- US-B1- 6 514 893

## Beschreibung

Die Erfindung betrifft Lithiumsilikat-Glaskeramiken und -Gläser mit hohem Gehalt an einem Element mit hoher Ordnungszahl, welche sich insbesondere zur Verwendung als Dentalmaterialien, beispielsweise zur Herstellung von Dentalrestaurationen eignen.

Lithiumsilikat-Glaskeramiken zeichnen sich durch sehr gute mechanische Eigenschaften aus, weshalb sie seit langem im Dentalbereich und dort vornehmlich zur Herstellung von Dentalkronen und kleinen Brücken Anwendung finden. Die bekannten Lithiumsilikat-Glaskeramiken enthalten üblicherweise als Hauptkomponenten SiO₂, Li₂O, Al₂O₃, Alkalimetall wie Na₂O oder K₂O und Keimbildner wie P₂O₅. Darüber hinaus können sie als weitere Komponenten beispielsweise weitere Alkalimetalloxide und/oder Erdalkalimetalloxide und/oder ZnO enthalten. Es sind auch Glaskeramiken bekannt, die in geringen Mengen weitere Metalloxide und insbesondere färbende und fluoreszierende Metalloxide enthalten.

EP 1 505 041 beschreibt Lithiumsilikat-Glaskeramiken, die zusätzlich 0 bis 2 Gew.-% ZrO₂ sowie 0,5 bis 7,5 Gew.-% und insbesondere 0,5 bis 3,5 Gew.-% färbende und fluoreszierende Metalloxide enthalten können. EP 1 688 398 beschreibt ähnliche Lithiumsilikat-Glaskeramiken, die im Wesentlichen frei von ZnO sind und neben färbenden und fluoreszierenden Metalloxiden in den vorgenannten Mengen auch 0 bis 4 Gew.-% ZrO₂ enthalten können, wobei zur Erzielung hoher Festigkeiten allerdings geringere Mengen von 0 bis 2 Gew.-% ZrO₂ bevorzugt sind. Die Glaskeramiken werden insbesondere in Form der Lithiummetasilikat-Glaskeramiken mittels CAD/CAM-Verfahren zu den gewünschten Dentalrestaurationen verarbeitet, wobei eine anschließende Wärmebehandlung die Umwandlung der Metasilikat-Phase in die hochfeste Disilikat-Phase bewirkt.

US 6,455,451 betrifft Lithiumdisilikat-Glaskeramiken, die neben anderen Komponenten auch Übergangsmetalloxide enthalten können. Dabei wird unter anderem vorgeschlagen, zur Erhöhung des Brechungsindex der Glasmatrix kleine Mengen schwerer Elemente wie Sr, Y, Nb, Cs, Ba, Ta, Ce, Eu oder Tb zuzugeben. So können beispielweise CeO₂ und Tb₄O₇ in Mengen 0 bis 1 Gew.-%, Nb₂O₃ und Ta₂O₅ in Mengen von 0 bis 2 Gew.-% und ZrO₂ und Y₂O₃ in Mengen von 0 bis 3 Gew.-% verwendet werden. In einer Ausführungsform soll Ta₂O₅ in einer Menge von 0,5 bis 8 Gew.-% vorliegen können, wobei die konkreten Beispiele allerdings maximal 2,02 Gew.-% dieses Oxids aufweisen.

US 5,176,961 und US 5,219,799 offenbaren Glaskeramiken beispielsweise zur Herstellung von Geschirr, die als Färbemittel bestimmte Übergangsmetalloxide wie CeO₂, Co₃O₄, Cr₂O₃, CuO, Fe₂O₃, MnO₂, NiO und V₂O₅ in einer Menge von 0,01 bis 7 Gew.-% enthalten können.

US 5,507,981 und US 5,702,514 beschreiben Verfahren zur Formung von Dentalrestaurationen und in diesen Verfahren einsetzbare Glaskeramiken. Dabei handelt es sich insbesondere um Lithiumdisilikat-Glaskeramiken, die 0 bis 5 Gew.-% färbende Oxide wie SnO₂, MnO, CeO, Fe₂O₃, Ni₂O, V₂O₃, Cr₂O₃ oder TiO₂ enthalten können.

Bekannte Glaskeramiken auf Basis von Lithiumsilikat weisen häufig optische Eigenschaften auf, die den ästhetischen Anforderungen insbesondere im Zusammenhang mit der Verwendung als Dentalmaterialien nicht in ausreichendem Maße genügen. So weisen bekannte Glaskeramiken oft einen ungünstigen Brechungsindex auf. Insbesondere besteht bei Glaskeramiken das Problem, dass die Brechungsindizes der kristallinen Phase und der Glasphase üblicherweise deutlich voneinander abweichen, was eine in den meisten Fällen unerwünschte Eintrübung der Glaskeramik zur Folge hat. Ähnliche Probleme bestehen etwa im Falle von Kompositen, weil die Brechungsindizes bekannter Glaskeramiken und Gläser üblicherweise von denen der Polymerphase abweichen. Es besteht daher ein Bedarf an Glaskeramiken auf Basis von Lithiumsilikat, deren Brechungsindex in einfacher Weise variiert werden kann, jedoch ohne dass dabei die sonstigen Eigenschaften wesentlich beeinträchtigt werden. Es ist zudem wünschenswert, dass solche Glaskeramiken unter vergleichbaren Bedingungen wie übliche Glaskeramiken hergestellt und kristallisiert werden können.

Diese Aufgabe wird durch die Lithiumsilikat-Dentalglaskeramik nach einem der Ansprüche 1 bis 11 und 14 gelöst. Gegenstand der Erfindung sind ebenfalls das Lithiumsilikat-Dentalglas nach einem der Ansprüche 12 bis 14, das Verfahren zur Herstellung der Glaskeramik und des Glases nach Anspruch 15 sowie deren Verwendung nach Anspruch 16.

Die erfindungsgemäße Lithiumsilikat-Dentalglaskeramik zeichnet sich dadurch aus, dass sie 54,0 bis 80,0 Gew.-% SiO₂ und 9,0 bis 30,0 Gew.-% Übergangsmetalloxid ausgewählt aus der Gruppe bestehend aus Oxiden von Yttrium, Oxiden von Übergangsmetallen mit Ordnungszahl von 41 bis 79 und Mischungen dieser Oxide enthält.

Generell ist es bevorzugt, dass das Übergangsmetalloxid als Komponente der erfindungsgemäßen Glaskeramik oder des erfindungsgemäßen Glases im Wesentlichen keine Farbänderung gegenüber einer entsprechenden Glaskeramik bzw. einem entsprechenden Glas ohne Zusatz dieser Komponente bewirkt. Insbesondere ist das Übergangsmetalloxid farblos und/oder nicht-fluoreszierend.

Vorzugsweise ist das Übergangsmetalloxid ausgewählt aus der Gruppe bestehend aus Oxiden von Y, Nb, La, Ta, W und Mischungen dieser Oxide.

Dabei sind Glaskeramiken bevorzugt, die 9,0 bis 25,0 Gew.-%, insbesondere 9,5 bis 20,0 Gew.-%, bevorzugt 10,0 bis 18,0 Gew.-%, weiter bevorzugt 10,5 bis 16,0 Gew.-% und am meisten bevorzugt 11,0 bis 15,0 Gew.-% Übergangsmetalloxid ausgewählt aus einer oder mehreren der vorgenannten Gruppen enthalten.

Überraschenderweise lässt sich durch die Verwendung des erfindungsgemäßen hohen Gehalts an Übergangsmetall mit hoher Ordnungszahl der Brechungsindex von Glaskeramiken und Gläsern auf Basis von Lithiumsilikat in einfacher Weise einstellen, ohne dass andere Eigenschaften wesentlich beeinträchtigt werden. Insbesondere hat sich unerwartet gezeigt, dass regelmäßig der hohe Gehalt an Übergangsmetall mit hoher Ordnungszahl weder die gewünschte Kristallisation von Lithiumdisilikat verhindert, noch zur Bildung unerwünschter Nebenkristallphasen führt, so dass erfindungsgemäß Glaskeramiken mit ausgezeichneten optischen und mechanischen Eigenschaften erhalten werden.

Bevorzugt ist weiter eine Glaskeramik, die 60,0 bis 70,0 Gew.-% SiO₂ enthält.

Außerdem ist eine Glaskeramik bevorzugt, die 11,0 bis 19,0 und insbesondere 12,0 bis 15,0 Gew.-% Li₂O enthält.

Es hat sich als besonders bevorzugt erwiesen, wenn die Glaskeramik 0,5 bis 12,0 und insbesondere 2,5 bis 6,0 Gew.-% Keimbildner enthält. Bevorzugte Keimbildner sind ausgewählt aus P₂O₅, TiO₂, Metallen, z.B. Pt, Pd, Au, Ag oder Mischungen davon. Besonders bevorzugt enthält die Glaskeramik P₂O₅ als Keimbildner. Überraschenderweise bewirkt insbesondere P₂O₅ als Keimbildner die Ausbildung von gewünschten Lithiumdisilikat-Kristallen und vermeidet auf der anderen Seite weitgehend die Bildung unerwünschter Nebenkristallphasen.

Die erfindungsgemäße Glaskeramik enthält bevorzugt weiteres Alkalimetalloxid in einer Menge von 0,5 bis 13,0, bevorzugt 1,0 bis 7,0 und besonders bevorzugt 2,0 bis 5,0 Gew.-%. Der Begriff "weiteres Alkalimetalloxid" bezeichnet Alkalimetalloxid mit Ausnahme von Li₂O. Das weitere Alkalimetalloxid ist insbesondere K₂O, Cs₂O und/oder Rb₂O und ist besonders bevorzugt K₂O. Es wird angenommen, dass der Einsatz von K₂O gegenüber dem in konventionellen Glaskeramiken eingesetzten Na₂O zur Verstärkung des Glasnetzwerkes beiträgt. Es ist bevorzugt, dass die Glaskeramik weniger als 2,0, insbesondere weniger als 1,0, bevorzugt weniger als 0,5 und besonders bevorzugt im Wesentlichen kein Na₂O enthält.

Weiter ist es bevorzugt, dass die Glaskeramik bis zu 6,0 Gew.-% und insbesondere 0,1 bis 5,0 Gew.-% Erdalkalimetalloxid enthält, wobei das Erdalkalimetalloxid insbesondere CaO, BaO, MgO, SrO oder eine Mischung davon ist.

Weiterhin ist es bevorzugt, dass die Glaskeramik bis zu 6,0 Gew.-% und insbesondere 0,1 bis 5,0 Gew.-% ZnO enthält.

Die erfindungsgemäße Glaskeramik kann darüber hinaus noch Zusatzkomponenten enthalten, die insbesondere ausgewählt sind aus Oxiden dreiwertiger Elemente, weiteren Oxiden vierwertiger Elemente, weiteren Oxiden fünfwertiger Elemente, Schmelzbeschleunigern, Färbemitteln und Fluoreszenzmitteln.

Bevorzugt ist eine Glaskeramik, die 0,2 bis 8,0, insbesondere 1,0 bis 7,0 und bevorzugt 2,5 bis 3,5 Gew.-% Oxid dreiwertiger Elemente enthält, wobei dieses Oxid insbesondere ausgewählt ist aus Al₂O₃, Bi₂O₃ und Mischungen davon, und bevorzugt Al₂O₃ ist.

Der Begriff "weitere Oxide vierwertiger Elemente" bezeichnet Oxide vierwertiger Elemente mit Ausnahme von SiO₂. Beispiele für weitere Oxide vierwertiger Elemente sind ZrO₂, SnO₂ und GeO₂ und insbesondere ZrO₂.

Der Begriff "weitere Oxide fünfwertiger Elemente" bezeichnet Oxide fünfwertiger Elemente mit Ausnahme von P₂O₅. Ein Beispiel für ein weiteres Oxid fünfwertiger Elemente ist Bi₂O₅.

Bevorzugt ist eine Glaskeramik, die mindestens ein weiteres Oxid vierwertiger Elemente oder ein weiteres Oxid fünfwertiger Elemente enthält.

Beispiele für Schmelzbeschleuniger sind Fluoride.

Beispiele für Färbemittel und Fluoreszenzmittel sind farbgebende oder fluoreszierende Oxide von d- und f-Elementen, wie z.B. die Oxide von Sc, Ti, Mn, Fe, Ag, Ce, Pr, Tb, Er und Yb, insbesondere Ti, Mn, Fe, Ag, Ce, Pr, Tb und Er.

Besonders bevorzugt ist eine Glaskeramik, die mindestens eine und bevorzugt alle folgenden Komponenten enthält:

| Komponente | Gew.-% |
|---|---|
| SiO₂ | 54,0 bis 80,0, insbesondere 60,0 bis 70,0 |
| Li₂O | 11,0 bis 19,0, insbesondere 12,0 bis 15,0 |
| K₂O | 0,5 bis 13,5, insbesondere 1,0 bis 7,0 |
| Al₂O₃ | 0,2 bis 8,0, insbesondere 1,0 bis 7,0 |
| Erdalkalioxid | 0 bis 6,0, insbesondere 0,1 bis 5,0 |
| ZnO | 0 bis 6,0, insbesondere 0,1 bis 5,0 |
| Übergangsmetalloxid | 9,0 bis 30,0, insbesondere 9,0 bis 25,0 |
| P₂O₅ | 0,5 bis 12,0, insbesondere 2,5 bis 6,0 |
| ZrO₂ | 0,1 bis 4,0, insbesondere 0,5 bis 2,0 |
| Färbemittel und Fluoreszenzmittel | 0,1 bis 8,0, insbesondere 0,2 bis 2,0. |

Der im Folgenden verwendete Begriff "Hauptkristallphase" bezeichnet die Kristallphase, die gegenüber anderen Kristallphasen den höchsten Volumenanteil hat.

Die erfindungsgemäße Glaskeramik weist bevorzugt Lithiummetasilikat als Hauptkristallphase auf. Insbesondere enthält die Glaskeramik mehr als 5 Vol.-%, bevorzugt mehr als 10 Vol.-% und besonders bevorzugt mehr als 15 Vol.-% an Lithiummetasilikat-Kristallen, bezogen auf die gesamte Glaskeramik.

In einer weiteren bevorzugten Ausführungsform weist die Glaskeramik Lithiumdisilikat als Hauptkristallphase auf. Insbesondere enthält die Glaskeramik mehr als 5 Vol.-%, bevorzugt mehr als 10 Vol.-% und besonders bevorzugt mehr als 15 Vol.-% an Lithiumdisilikat-Kristallen, bezogen auf die gesamte Glaskeramik.

Die erfindungsgemäße Lithiumdisilikat-Glaskeramik zeichnet sich durch besonders gute mechanische Eigenschaften aus und kann durch Wärmebehandlung der erfindungsgemäßen Lithiummetasilikat-Glaskeramik erzeugt werden.

Auch ist es überraschend, dass die erfindungsgemäße Lithiumdisilikat-Glaskeramik trotz ihres hohen Gehalts an Oxid eines Übergangsmetalls mit hoher Ordnungszahl regelmäßig eine gute Transluzenz besitzt und bei ihr keine Amorph-Amorph-Phasentrennung auftritt und sie damit beispielsweise zur ästhetisch ansprechenden Beschichtung von dentalen Restaurationen eingesetzt werden kann.

Die erfindungsgemäße Lithiumdisilikat-Glaskeramik hat gute mechanische Eigenschaften und eine hohe chemische Beständigkeit.

Die Erfindung betrifft ebenfalls ein Lithiumsilikat-Dentalglas, das 54,0 bis 80,0 Gew.-% SiO₂, 11,0 bis 19,0 Gew.-% Li₂O und 9,0 bis 30,0 Gew.-% Übergangsmetalloxid ausgewählt aus der Gruppe bestehend aus Oxiden von Yttrium, Oxiden von Übergangsmetallen mit Ordnungszahl von 41 bis 79 und Mischungen dieser Oxide enthält. Hinsichtlich bevorzugter Ausführungsformen dieses Glases wird auf die oben beschriebenen bevorzugten Ausführungsformen der erfindungsgemäßen Glaskeramik verwiesen. Es hat sich überraschend gezeigt, dass trotz des hohen Gehalts an Übergangsmetall mit hoher Ordnungszahl homogene, klare Gläser erhalten werden können, die keinerlei unerwünschte Phänomene wie Amorph-Amorph-Phasentrennung oder Spontankristallisation zeigen. Diese Gläser sind daher zur Herstellung der erfindungsgemäßen Glaskeramik geeignet. Alternativ ist auch eine Verwendung beispielsweise als Füllstoff etwa in Dentalmaterialien, insbesondere anorganisch-organischen Kompositen möglich. Gegenstand der Erfindung ist auch eine polymerisierbare Zusammensetzung, die eine Glaskeramik oder ein Glas wie oben beschrieben und mindestens ein polymerisierbares Monomer enthält. Geeignete Monomere und weitere Bestandteile von Kompositen sind dem Fachmann bekannt.

Besonders bevorzugt ist ein Lithiumsilikatglas mit Keimen, die zur Ausbildung von Lithiummetasilikat- und/oder Lithiumdisilikat-kristallen geeignet sind.

Das erfindungsgemäße Glas mit Keimen kann durch Wärmebehandlung eines entsprechend zusammengesetzten Ausgangsglases erzeugt werden. Durch eine weitere Wärmebehandlung kann dann die erfindungsgemäße Lithiummetasilikat-Glaskeramik gebildet werden, die ihrerseits durch weitere Wärmebehandlung in die erfindungsgemäße Lithiumdisilikat-Glaskeramik umgewandelt werden kann. Mithin können das Ausgangsglas, das Glas mit Keimen und die Lithiummetasilikat-Glaskeramik als Vorstufen zur Erzeugung der hochfesten Lithiumdisilikat-Glaskeramik angesehen werden.

Die erfindungsgemäße Glaskeramik und das erfindungsgemäße Glas liegen insbesondere in Form von Pulvern oder Rohlingen vor, da sie in diesen Formen einfach weiterverarbeitet werden können. Sie können aber auch in Form von dentalen Restaurationen, wie Inlays, Onlays, Kronen oder Abutments vorliegen.

Die Erfindung betrifft ebenfalls ein Verfahren zur Herstellung der erfindungsgemäßen Glaskeramik und des erfindungsgemäßen Glases mit Keimen, bei dem ein Ausgangsglas mit den Komponenten der Glaskeramik oder des Glases mindestens einer Wärmebehandlung im Bereich von 450 bis 950 °C unterzogen wird.

Das Ausgangsglas enthält daher 9,0 bis 30,0 Gew.-% Oxid von mindestens einem Übergangsmetall wie oben definiert. Darüber hinaus enthält es bevorzugt auch geeignete Mengen an SiO₂ und Li₂O, um die Ausbildung einer Lithiumsilikat-Glaskeramik zu ermöglichen. Weiter kann das Ausgangsglas auch noch andere Komponenten enthalten, wie sie oben für die erfindungsgemäße Lithiumsilikat-Glaskeramik angegeben sind. Dabei sind solche Ausführungsformen bevorzugt, die auch für die Glaskeramik als bevorzugt angegeben sind.

Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600 °C, vorzugsweise 1450 bis 1500 °C, für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen gegossen werden, um Rohlinge des Ausgangsglases, sogenannte Massivglasrohlinge oder monolithische Rohlinge, zu erzeugen. Vorzugsweise erfolgt die Abkühlung ab einer Temperatur von 500 °C mit einer Abkühlgeschwindigkeit von 3 bis 5 K/min bis auf Raumtemperatur. Dies ist insbesondere zur Erzeugung spannungsfreier Glasprodukte vorteilhaft.

Es ist ebenfalls möglich, die Schmelze erneut in Wasser zu geben, um ein Granulat herzustellen. Dieses Granulat kann dann nach Mahlen und gegebenenfalls Zugabe weiterer Komponenten, wie Färbe- und Fluoreszenzmitteln, zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung im Bereich von 450 bis 950 °C unterzogen. Es ist bevorzugt, dass zunächst bei einer Temperatur im Bereich von 500 bis 600 °C eine erste Wärmebehandlung durchgeführt wird, um ein erfindungsgemäßes Glas mit Keimen herzustellen, welche zur Bildung von Lithiummetasilikat- und/oder Lithiumdisilikat-Kristallen geeignet sind. Dieses Glas kann dann bevorzugt mindestens einer weiteren Temperaturbehandlung bei einer höheren Temperatur und insbesondere mehr als 570 °C unterworfen werden, um Kristallisation von Lithiummetasilikat oder von Lithiumdisilikat zu bewirken.

Die im erfindungsgemäßen Verfahren durchgeführte mindestens eine Wärmebehandlung kann auch im Rahmen des Aufpressens oder Aufsinterns des erfindungsgemäßen Glases oder der erfindungsgemäßen Glaskeramik auf eine Keramik erfolgen.

Aus der erfindungsgemäßen Glaskeramik und dem erfindungsgemäßen Glas können dentale Restaurationen, wie Inlays, Onlays, Kronen oder Abutments, hergestellt werden. Die Erfindung betrifft daher auch deren Verwendung zur Herstellung dentaler Restaurationen.

Aufgrund der vorstehend geschilderten Eigenschaften der erfindungsgemäßen Glaskeramik und des erfindungsgemäßen Glases als dessen Vorläufer eignen sich diese insbesondere auch zum Einsatz in der Zahnheilkunde. Gegenstand der Erfindung ist daher auch die Verwendung der erfindungsgemäßen Glaskeramik oder des erfindungsgemäßen Glases als Dentalmaterial und insbesondere zur Herstellung dentaler Restaurationen oder als Beschichtungsmaterial für dentale Restaurationen, wie Kronen und Brücken.

Die Erfindung wird im Folgenden anhand von Beispielen näher erläutert.

### Beispiele

### Beispiele 1 bis 9 - Zusammensetzung und Kristallphasen

Es wurden insgesamt 9 Gläser und Glaskeramiken mit der in Tabelle I angegebenen Zusammensetzung (jeweils in Gew.-%) über Erschmelzung entsprechender Ausgangsgläser und anschließende Wärmebehandlung zur gesteuerten Keimbildung und Kristallisation hergestellt.

Die Ausgangsgläser wurden zunächst im 100 bis 200 g Maßstab aus üblichen Rohstoffen bei 1400 bis 1500 °C erschmolzen und durch Eingießen in Wasser in Glasfritten umgewandelt. Diese Glasfritten wurden zur Homogenisierung anschließend ein zweites Mal bei 1450 bis 1550 °C für 1 bis 3 h geschmolzen. Die erhaltenen Glasschmelzen wurden in vorgewärmte Formen gegossen, um Glasmonolithe zu erzeugen. Diese Glasmonolithe wurden durch thermische Behandlung zu erfindungsgemäßen Gläsern und Glaskeramiken umgewandelt.

Die nach Abschluss aller Wärmebehandlungen erhaltenen Kristallphasen wurden durch Hochtemperatur-Röntgenbeugung (HT-XRD) bei den jeweils in Tabelle I aufgeführten Temperaturen ermittelt. Es wurden überraschenderweise stets Glaskeramiken mit Lithiumdisilikat als Hauptkristallphase erhalten. Trotz des hohen Gehaltes an Übergangsmetallen mit hoher Ordnungszahl wurden keine Nebenkristallphasen mit diesen Übergangsmetallen gefunden.

Schließlich wurden die Brechungsindizes der jeweiligen Glasphasen mittels Abbe-Refraktometrie (20 °C, 589 nm) bestimmt. Dabei zeigte sich, dass die erfindungsgemäßen Glaskeramiken einen deutlich höheren Brechungsindex als die Vergleichs-Glaskeramik aufwiesen.

**Tabelle I**

| | **1** | **2** | **3** | **4** | **5** | **6** | **7** | **8** | **9** |
|---|---|---|---|---|---|---|---|---|---|
| **SiO₂** | 58,4 | 66,4 | 63,5 | 67,0 | 61,8 | 66,4 | 66,4 | 61,8 | 54,5 |
| **K₂O** | 1,0 | 2,9 | 2,8 | 2,9 | 1,0 | 2,9 | 2,9 | 1,0 | 0,5 |
| **Li₂O** | 12,1 | 13,8 | 13,2 | 14,4 | 13,2 | 13,8 | 13,8 | 13,2 | 11,3 |
| **Al₂O₃** | 1,0 | 2,9 | 2,5 | | 1,0 | 2,9 | 2,9 | 1,0 | 0,5 |
| **P₂O₅** | 2,5 | 4,0 | 4,0 | 4,0 | 5,0 | 4,0 | 4,0 | 5,0 | 3,2 |
| **WO₃** | | | | | | | | | |
| **Nb₂O₅** | | 10,0 | | | | | | | |
| **Ta₂O₅** | | | | | | 10,0 | | | |
| **La₂O₃** | 25,0 | | | | | | 10,0 | 18,0 | 30,0 |
| **Y₂O₃** | | | 14,0 | 10,0 | 18,0 | | | | |
| **CeO₂** | | | | 1,0 | | | | | |
| **Er₂O₃** | | | | 0,3 | | | | | |
| **Tb₄O₇** | | | | 0,4 | | | | | |
| **Summe** | 100,0 | 100,0 | 100,0 | 100,0 | 100,0 | 100,0 | 100,0 | 100,0 | |
| **Kristallphase (n) HT-XRD** | | Li₂Si₂O₅ (800°C) | Li₂Si₂O₅ KAlSiO₄ (820°C) | Li₂Si₂O₅ (780°C) | Li₂Si₂O₅ Li₂SiO₃ (800°C) | Li₂Si₂O₅ (800°C) | Li₂Si₂O₅ Li₂SiO₃ (700°C) | Li₂Si₂O₅ LaPO₄ (800°C) | |
| **Brechungs index n_{d}** | | 1,5547 | 1,5553 | 1,5494 | 1,5643 | 1,5403 | 1,5422 | 1,5586 | |

## Patentansprüche

1. Lithiumsilikat-Dentalglaskeramik, die 54,0 bis 80,0 Gew.-% SiO₂ und 9,0 bis 30,0 Gew.-% Übergangsmetalloxid ausgewählt aus der Gruppe bestehend aus Oxiden von Yttrium, Oxiden von Übergangsmetallen mit Ordnungszahl von 41 bis 79 und Mischungen dieser Oxide enthält.

2. Glaskeramik nach Anspruch 1, die 9,0 bis 30,0 Gew.-% Übergangsmetalloxid ausgewählt aus der Gruppe bestehend aus Oxiden von Y, Nb, La, Ta, W und Mischungen dieser Oxide enthält.

3. Glaskeramik nach Anspruch 1 oder 2, die 9,0 bis 25,0 Gew.-%, insbesondere 9,5 bis 20,0 Gew.-%, bevorzugt 10,0 bis 18,0 Gew.-%, weiter bevorzugt 10,5 bis 16,0 Gew.-% und am meisten bevorzugt 11,0 bis 15,0 Gew.-% Übergangsmetalloxid enthält.

4. Glaskeramik nach einem der Ansprüche 1 bis 3, die 60,0 bis 70,0 Gew.-% SiO₂ enthält.

5. Glaskeramik nach einem der Ansprüche 1 bis 4, die 11,0 bis 19,0 und insbesondere 12,0 bis 15,0 Gew.-% Li₂O enthält.

6. Glaskeramik nach einem der Ansprüche 1 bis 5, die 0,5 bis 12,0 und insbesondere 2,5 bis 6,0 Gew.-% Keimbildner enthält, wobei der Keimbildner insbesondere ausgewählt ist aus P₂O₅, TiO₂ und/oder Metallen und bevorzugt P₂O₅ ist.

7. Glaskeramik nach einem der Ansprüche 1 bis 6, die weiteres Alkalimetalloxid in einer Menge von 0,5 bis 13,5, bevorzugt 1,0 bis 7,0 und besonders bevorzugt 2,0 bis 5,0 Gew.-% enthält, wobei das weitere Alkalimetalloxid insbesondere K₂O, Cs₂O und/oder Rb₂O ist und bevorzugt K₂O ist.

8. Glaskeramik nach einem der Ansprüche 1 bis 7, die bis zu 6,0 Gew.-% und insbesondere 0,1 bis 5,0 Gew.-% Erdalkalimetalloxid enthält, wobei das Erdalkalimetalloxid bevorzugt CaO, BaO, MgO und/oder SrO ist, und/oder bis zu 6,0 Gew.-% und insbesondere 0,1 bis 5,0 Gew.-% ZnO enthält.

9. Glaskeramik nach einem der Ansprüche 1 bis 8, die 0,2 bis 8,0, insbesondere 1,0 bis 7,0 und bevorzugt 2,5 bis 3,5 Gew.-% Oxid dreiwertiger Elemente enthält, wobei das Oxid dreiwertiger Elemente insbesondere Al₂O₃ und/oder Bi₂O₃ ist und bevorzugt Al₂O₃ ist.

10. Glaskeramik nach einem der Ansprüche 1 bis 9, die mindestens ein weiteres Oxid vierwertiger Elemente, insbesondere ZrO₂, SnO₂ oder GeO₂, oder ein weiteres Oxid fünfwertiger Elemente, insbesondere Bi₂O₅, enthält.

11. Glaskeramik nach einem der Ansprüche 1 bis 10, die mindestens eine und bevorzugt alle folgenden Komponenten enthält:
| Komponente | Gew.-% |
|---|---|
| SiO₂ | 54,0 bis 80,0, insbesondere 60,0 bis 70,0 |
| Li₂O | 11,0 bis 19,0, insbesondere 13,0 bis 17,0 |
| Al₂O₃ | 0,2 bis 8,0, insbesondere 1,0 bis 7,0 |
| K₂O | 0,5 bis 13,5, insbesondere 1,0 bis 7,0 |
| Erdalkalioxid | 0 bis 6,0, insbesondere 0,1 bis 5,0 |
| ZnO | 0 bis 6,0, insbesondere 0,1 bis 5,0 |
| Übergangsmetalloxid | 9,0 bis 30,0, insbesondere 9,0 bis 25,0 |
| P₂O₅ | 0,5 bis 12,0, insbesondere 2,5 bis 6,0 |
| ZrO₂ | 0,1 bis 4,0, insbesondere 0,5 bis 2,0. |

12. Lithiumsilikat-Dentalglas, das 54,0 bis 80,0 Gew.-% SiO₂, 11,0 bis 19,0 Gew.-% Li₂O und 9,0 bis 30,0 Gew.-% Übergangsmetalloxid ausgewählt aus der Gruppe bestehend aus Oxiden von Yttrium, Oxiden von Übergangsmetallen mit Ordnungszahl von 41 bis 79 und Mischungen dieser Oxide enthält.

13. Glas nach Anspruch 12, wobei das Glas Keime enthält, die zur Ausbildung von Lithiummetasilikat- und/oder Lithiumdisilikatkristallen geeignet sind.

14. Glaskeramik nach einem der Ansprüche 1 bis 11 oder Glas nach Anspruch 12 oder 13, welche in Form von einem Pulver, einem Rohling oder einer dentalen Restauration vorliegen.

15. Verfahren zu Herstellung der Glaskeramik nach einem der Ansprüche 1 bis 11 oder 14 oder des Glases nach einem der Ansprüche 12 bis 14, bei dem ein Ausgangsglas mit den Komponenten der Glaskeramik oder des Glases mindestens einer Wärmebehandlung im Bereich von 450 bis 950 °C unterzogen wird.

16. Verwendung der Glaskeramik nach einem der Ansprüche 1 bis 11 oder 14 oder des Glases nach einem der Ansprüche 12 bis 14 als Dentalmaterial und insbesondere zur Beschichtung dentaler Restaurationen und zur Herstellung dentaler Restaurationen und als Füllstoff in anorganisch-organischen Kompositen.

## Claims

1. Lithium silicate dental glass ceramic, which comprises 54.0 to 80.0 wt.-% SiO₂ and 9.0 to 30.0 wt.-% transition metal oxide selected from the group consisting of oxides of yttrium, oxides of transition metals with an atomic number from 41 to 79 and mixtures of these oxides.

2. Glass ceramic according to claim 1, which comprises 9.0 to 30.0 wt.-% transition metal oxide selected from the group consisting of oxides of Y, Nb, La, Ta, W and mixtures of these oxides.

3. Glass ceramic according to claim 1 or 2, which comprises 9.0 to 25.0 wt.-%, in particular 9.5 to 20.0 wt.-%, preferred 10.0 to 18.0 wt.-%, more preferred 10.5 to 16.0 wt.-% and most preferred 11.0 to 15.0 wt.-% of the transition metal oxide.

4. Glass ceramic according to any one of claims 1 to 3, which comprises 60.0 to 70.0 wt.-% SiO₂.

5. Glass ceramic according to any one of claims 1 to 4, which comprises 11.0 to 19.0 and in particular 12.0 to 15.0 wt.-% Li₂O.

6. Glass ceramic according to any one of claims 1 to 5, which comprises 0.5 to 12.0 and in particular 2.5 to 6.0 wt.-% nucleating agent, wherein the nucleating agent is selected in particular from P₂O₅, TiO₂, and/or metals and preferably is P₂O₅.

7. Glass ceramic according to any one of claims 1 to 6, which comprises further alkali metal oxide in an amount of from 0.5 to 13.5, preferably 1.0 to 7.0 and particularly preferably 2.0 to 5.0 wt.-%, wherein the further alkali metal oxide is in particular K₂O, Cs₂O and/or Rb₂O and preferably is K₂O.

8. Glass ceramic according to any one of claims 1 to 7, which comprises up to 6.0 wt.-% and in particular 0.1 to 5.0 wt.-% alkaline earth metal oxide, wherein the alkaline earth metal oxide is preferably CaO, BaO, MgO and/or SrO, and/or comprises up to 6.0 wt.-% and in particular 0.1 to 5.0 wt.-% ZnO.

9. Glass ceramic according to any one of claims 1 to 8, which comprises 0.2 to 8.0, in particular 1.0 to 7.0 and preferably 2.5 to 3.5 wt.-% oxide of trivalent elements, wherein the oxide of trivalent elements is in particular Al₂O₃ and/or Bi₂O₃ and preferably is Al₂O₃.

10. Glass ceramic according to any one of claims 1 to 9, which comprises at least one further oxide of tetravalent elements, in particular ZrO₂, SnO₂ or GeO₂, or at least one further oxide of pentavalent elements, in particular Bi₂O₅.

11. Glass ceramic according to any one of claims 1 to 10, which comprises at least one and preferably all of the following components:
| Component | wt.-% |
|---|---|
| SiO₂ | 54.0 to 80.0, in particular 60.0 to 70.0 |
| Li₂O | 11.0 to 19.0, in particular 13.0 to 17.0 |
| Al₂O₃ | 0.2 to 8.0, in particular 1.0 to 7.0 |
| K₂O | 0.5 to 13.5, in particular 1.0 to 7.0 |
| Alkaline earth oxide | 0 to 6.0, in particular 0.1 to 5.0 |
| ZnO | 0 to 6.0, in particular 0.1 to 5.0 |
| Transition metal oxide | 9.0 to 30.0, in particular 9.0 to 25.0 |
| P₂O₅ | 0.5 to 12.0, in particular 2.5 to 6.0 |
| ZrO₂ | 0.1 to 4.0, in particular 0.5 to 2.0. |

12. Lithium silicate dental glass, which comprises 54.0 to 80.0 wt.-% SiO₂, 11.0 to 19.0 wt.-% Li₂O and 9.0 to 30.0 wt.-% transition metal oxide selected from the group consisting of oxides of yttrium, oxides of transition metals with an atomic number from 41 to 79 and mixtures of these oxides.

13. Glass according to claim 12, wherein the glass comprises nuclei which are suitable for forming lithium metasilicate and/or lithium disilicate crystals.

14. Glass ceramic according to any one of claims 1 to 11 or glass according to claim 12 or 13, which are present in the form of a powder, a blank or a dental restoration.

15. Process for the preparation of the glass ceramic according to any one of claims 1 to 11 or 14 or of the glass according to any one of claims 12 to 14, in which a starting glass with the components of the glass ceramic or the glass is subjected to at least one heat treatment in the range of from 450 to 950°C.

16. Use of the glass ceramic according to any one of claims 1 to 11 or 14 or of the glass according to any one of claims 12 to 14 as dental material and in particular for coating dental restorations and for the preparation of dental restorations and as filler in inorganic-organic composites.

## Revendications

1. Vitrocéramique dentaire à base de silicate de lithium, qui contient 54,0 à 80,0 % en poids de SiO₂ et 9,0 à 30,0 % en poids d'oxyde de métal de transition, sélectionné dans le groupe comprenant des oxydes d'yttrium, des oxydes de métaux de transition de numéro d'ordre allant de 41 à 79 et des mélanges de ces oxydes.

2. Vitrocéramique selon la revendication 1, qui contient 9,0 à 30,0 % en poids d'oxyde de métal de transition, sélectionné dans le groupe comprenant des oxydes de Y, Nb, La, Ta, W et des mélanges de ces oxydes.

3. Vitrocéramique selon la revendication 1 ou 2, qui contient 9,0 à 25,0 % en poids, notamment 9,5 à 20,0 % en poids, de préférence 10,0 à 18,0 % en poids, plus particulièrement 10,5 à 16,0 % en poids et de façon particulièrement avantageuse 11,0 à 15,0 % en poids d'oxyde de métal de transition.

4. Vitrocéramique selon l'une des revendications 1 à 3, qui contient 60,0 à 70,0 % en poids de SiO₂.

5. Vitrocéramique selon l'une des revendications 1 à 4, qui contient 11,0 à 19,0 et notamment 12,0 à 15,0 % en poids de LiO₂.

6. Vitrocéramique selon l'une des revendications 1 à 5, qui contient 0,5 à 12,0 et notamment 2,5 à 6,0 % en poids d'agent de nucléation, l'agent de nucléation étant notamment sélectionné parmi P₂O₅, TiO₂ et/ou des métaux et est de préférence P₂O₅.

7. Vitrocéramique selon l'une des revendications 1 à 6, qui contient de l'oxyde de métal alcalin supplémentaire en une quantité allant de 0,5 à 13,5, de préférence de 1,0 à 7,0 et de façon particulièrement avantageuse de 2,0 à 5,0 % en poids, l'oxyde de métal alcalin supplémentaire étant notamment K₂O, Cs₂O et/ou Rb₂O et est de préférence K₂O.

8. Vitrocéramique selon l'une des revendications 1 à 7, qui contient jusqu'à 6,0 % en poids et notamment 0,1 à 5,0 % en poids d'oxyde de métal alcalinoterreux, l'oxyde de métal alcalinoterreux étant de préférence CaO, BaO, MgO et/ou SrO, et/ou contient jusqu'à 6,0 % en poids et notamment 0,1 à 5,0 % en poids de ZnO.

9. Vitrocéramique selon l'une des revendications 1 à 8, qui contient 0,2 % à 8,0, notamment 1,0 à 7,0 et de préférence 2,5 à 3,5 % en poids d'oxyde d'éléments trivalents, l'oxyde d'éléments trivalents étant notamment Al₂O₃ et/ou Bi₂O₃ et est de préférence Al₂O₃.

10. Vitrocéramique selon l'une des revendications 1 à 9, qui contient au moins un autre oxyde d'éléments tétravalents, notamment ZrO₂, SnO₂ ou GeO₂, ou un autre oxyde d'éléments pentavalents, notamment Bi₂O₅.

11. Vitrocéramique selon l'une des revendications 1 à 10, qui contient au moins un et de préférence tous les constituants suivants :
| Constituant | % en poids |
|---|---|
| SiO₂ | 54,0 à 80,0, notamment 60,0 à 70,0 |
| Li₂O | 11,0 à 19,0, notamment 13,0 à 17,0 |
| Al₂O₃ | 0,2 à 8,0, notamment 1,0 à 7,0 |
| K₂O | 0,5 à 13,5, notamment 1,0 à 7,0 |
| Oxyde de métal alcalinoterreux | 0 à 6,0, notamment 0,1 à 5,0 |
| ZnO | 0 à 6,0, notamment 0,1 à 5,0 |
| Oxyde de métal de transition | 9,0 à 30,0, notamment 9,0 à 25,0 |
| P₂O₅ | 0,5 à 12,0, notamment 2,5 à 6,0 |
| ZrO₂ | 0,1 à 4,0, notamment 0,5 à 2,0. |

12. Verre dentaire à base de silicate de lithium, qui contient 54,0 à 80,0 ˙˙% en poids de SiO₂, 11,0 à 19,0 % en poids de Li₂O et 9,0 à 30,0 % en poids d'oxyde de métal de transition, sélectionné dans le groupe comprenant des oxydes d'yttrium, des oxydes de métaux de transition de numéro atomique allant de 41 à 79 et des mélanges de ces oxydes.

13. Verre selon la revendication 12, le verre contenant des germes qui sont aptes à former des cristaux de métasilicate de lithium et/ou de disilicate de lithium.

14. Vitrocéramique selon l'une des revendications 1 à 11 ou verre selon la revendication 12 ou 13, qui sont présents sous la forme d'une poudre, d'une ébauche ou d'une restauration dentaire.

15. Procédé de fabrication de la vitrocéramique selon l'une des revendications 1 à 11 ou 14 ou du verre selon l'une des revendications 12 à 14, selon lequel un verre de départ est soumis avec les constituants de la vitrocéramique ou du verre à au moins un traitement thermique dans la plage allant de 450 à 950 °C.

16. Utilisation de la vitrocéramique selon l'une des revendications 1 à 11 ou 14 ou du verre selon l'une des revendications 12 à 14, en tant que matériau dentaire et notamment pour recouvrir des restaurations dentaires et pour fabriquer des restaurations dentaires et en tant que matériau de remplissage dans des composites inorganiques-organiques.
